# EUROPEAN PATENT APPLICATION

(11) **EP 4 253 555 A1**
(43) Date of publication of application: **04.10.2023**
(21) Application number: 21903700.9
(22) Date of filing: 25.11.2021
(51) Int. Cl.: C12Q 1/04, C12N 5/00

(54) **PLASTIC-DEGRADING MICROORGANISM SCREENING MEDIUM AND MANUFACTURING METHOD THEREFOR**

(30) Priority: 08.12.2020 KR 20200170235
(71) Applicant: Korea Research Institute of Chemical Technology, Daejeon 34114 (KR)
(72) Inventor: OH, Dong Yeop, Daejeon 34114 (KR); PARK, Jeyoung, Daejeon 34114 (KR); HWANG, Sung Yeon, Daejeon 34114 (KR); SHIN, Gi Young, Daejeon 34114 (KR); KOO, Jun Mo, Daejeon 34114 (KR); JEON, Hyeon Yeol, Daejeon 34114 (KR); CHOI, Sejin, Daejeon 34114 (KR)
(74) Representative: Behr, Wolfgang
(86) International application number: PCT/KR2021/017499
(87) International publication number: WO 2022/124654

(57) **Abstract**

The purpose of the present invention is to prepare a plastic-degrading microorganism screening medium. Specifically, provided is a plastic-degrading microorganism screening medium in which an island-type polymer layer or a mesh-type polymer layer is formed on a solid medium to allow a polymer clear zone or halo zone, formed due to the plastic degrading activity of microorganisms, to be easily, visually identified.

## Description

### [Technical Field]

The present invention relates to production of a plastic-degrading microorganism screening medium. In particular, the present invention relates to provision of a plastic-degrading microorganism screening medium which allows a polymer clear zone or halo zone formed by the plastic degrading activity of microorganisms to be easily visually confirmed.

### [Background Art]

Since plastic wastes currently entering the environment are not decomposed in nature, ecosystem destruction due to accumulation of the plastic is deepening, and thus, biodegradation studies on all types of plastics including biodegradable, hardly-degradable, and nondegradable polymers are in progress.

In addition, in order to understand the influence of the surrounding exposed environment such as marine, soil, or fresh water and the influence of degradation of an additive supplemented for reinforcing plastic properties, and the like, discovery of various plastic-degrading microorganisms in the natural environment should be preceded.

As such, as a method of discovering microorganisms degrading the plastic, a method of screening a plastic-degrading microorganism among numerous microorganisms which grow in a culture environment including plastic may be used.

However, since plastics are generally insoluble in water, it is difficult to produce a solid medium in which plastics are uniformly distributed by a method of simply adding them to a medium composition.

In addition, there is a limit to crushing plastics to prepare fine powder, and also it is difficult to mix them uniformly.

In the case of a method of producing the plastic in a film form and incubating it in a solid medium or a liquid medium to figure out a degradation degree or to detect microorganisms attached to the surface, it takes a considerable time to determine the results, and in particular, there is a possibility of causing a misreading, so that screening efficiency is low.

Meanwhile, a mixing method using a plastic emulsion may be introduced for solving the problems, but it is difficult to establish an appropriate emulsion production method for each polymer. In addition, since the emulsion production step is also complicated, it is difficult to establish emulsion conditions, and thus, there is a limit to using the method as a method for producing a medium for screening.

Therefore, under the background, the development of a method of producing a medium for effectively screening plastic-degrading microorganisms is being demanded.

### [Disclosure]

### [Technical Problem]

An object of the present invention is to provide a method of screening plastic-degrading microorganisms, which includes producing a solid medium including plastics by a simple method and effectively discovering microorganisms which may degrade plastics from the natural environment, through the solid medium.

Recognizing the complexity of a production step of a medium produced by a conventional mixing method using a plastic emulsion and the difficulty in establishing emulsion conditions, another object of the present invention is to provide a plastic-degrading microorganism screening medium of a new method for solving the problem.

Still another object of the present invention is to provide a plastic-degrading microorganism screening medium which may screen microorganisms degrading plastics more clearly than a conventional method.

### [Technical Solution]

In one general aspect, a plastic-degrading microorganism screening medium includes a medium, and an island-like or mesh-like polymer layer on the medium.

The plastic-degrading microorganism screening medium according to an exemplary embodiment of the present invention allows observation of degradation activity of the polymer layer, by applying microorganisms degrading a polymer on the polymer layer.

The island-like polymer layer or the mesh-like polymer layer according to an exemplary embodiment of the present invention may be produced by spraying through an atomization device.

The atomization device according to an exemplary embodiment of the present invention may be selected from an atomizer, a sprayer, an air brush, or an electrospinning device.

According to an exemplary embodiment of the present invention, the island-like polymer layer may have a particle diameter of 500 um or less and the mesh-like polymer layer may have a fiber diameter of 200 um or less.

In another general aspect, a method of producing a plastic-degrading microorganism screening medium includes: spraying a plastic solution on a sterilized base solid medium to form an island-like or mesh-like polymer layer on the solid medium.

The solid medium according to an exemplary embodiment of the present invention may include one or more selected from the group consisting of agar, gelatin, silica gel, serum, and albumin.

The plastic solution according to an exemplary embodiment of the present invention may have a solid content of 0.01 to 20 wt%.

The spraying according to an exemplary embodiment of the present invention may include spraying using an electrospinning device or spraying equipment including a coaxial double or multiple nozzle part and an air compression part.

According to an exemplary embodiment of the present invention, a voltage of the electrospinning device may be 1 to 60 kV, and a discharge speed of the plastic solution may be 0.5 to 100 ml/h.

### [Advantageous Effects]

Since the plastic-degrading microorganism screening medium according to the present invention may be produced regardless of the composition of the solid medium, the medium may be useful for screening under various culture conditions by adjusting a medium composition.

The plastic-degrading microorganism screening medium according to the present invention is produced by a simple method, allows mass production of plastic-degrading microorganism screening media having a certain standard, and is easily used as a basic material for identification and research and commercialization of plastic-degrading microorganisms.

Using the method of producing a solid medium and the screening method according to the present invention, microorganisms having plastic degrading activity cause a visually recognizable change while being degraded for using plastic particles on a solid medium surface as a nutrient in growth of microorganisms having plastic degrading activity, and thus, screening may be done more efficiently.

### [Description of Drawings]

FIG. 1 is a photograph of a plastic-degrading microorganism screening medium obtained by applying plastic particles on the agar solid medium using a pneumatic spraying method, according to an exemplary embodiment of the present invention, in which the left half circle is a photograph before spraying and the right half circle is a photograph after spraying the plastic particles.
FIG. 2 is photographs of an island-like polymer layer applied on the plastic-degrading microorganism screening medium of Example 1, which were taken by an electron microscope.
FIG. 3 is a photograph of a result in which a clear zone was formed around a colony as microorganisms grow in the plastic-degrading microorganism screening medium of Example 1.
FIG. 4 is photographs of a result of culture on an agar medium mixed with crushed plastic of Comparative Example 1 and a result of culture in an agar medium in which a plastic film is bonded on an agar medium of Comparative Example 2.

### [Best Mode]

Hereinafter, the present invention will be described in more detail with reference to specific examples and exemplary embodiments including the accompanying drawings. However, the following specific examples or exemplary embodiments are only a reference for describing the present invention in detail, and the present invention is not limited thereto, and may be implemented in various forms.

In addition, unless otherwise defined, all technical terms and scientific terms have the same meanings as those commonly understood by one of those skilled in the art to which the present invention pertains. The terms used herein are only for effectively describing a certain specific example, and are not intended to limit the present invention.

In addition, the singular form used in the specification and claims appended thereto may be intended to include a plural form also, unless otherwise indicated in the context.

In addition, unless particularly described to the contrary, "comprising" any elements will be understood to imply further inclusion of other elements rather than the exclusion of any other elements.

In addition, a "plastic" described in the present invention is the same material as a "polymer".

The term "medium" in the present invention refers to a mixture including a nutritional material required for growth of microorganisms for incubating microorganisms, and an additional material may be added depending on the purpose. The medium used in the present invention includes all of a natural medium, a synthetic medium, and a selected medium, and may be any medium as long as it is used in common microorganism culture.

Conventionally, for screening plastic-degrading microorganisms, a solid medium mixed with plastic fine powder, or a solid medium produced by preparing a plastic solution in an emulsion form and mixing the solution with a medium is used to screen microorganisms.

However, when the medium is produced by mixing with plastic fine powder, the plastic powder is precipitated and agglomerated during solidification, and during microorganism screening, it is difficult to identify a clear zone or a halo zone to allow confirmation of the corresponding microorganism.

A microorganism screening medium may be produced by preparing a plastic solution in an emulsion form and mixing the emulsion with the medium, but the conditions for preparing the emulsion are difficult.

In addition, the method has difficulties in the changed conditions of the emulsion depending on the type of plastics and the type of solvent in which the plastics are dissolved, and there is a difficulty in setting new conditions for each experiment.

Thus, a method of producing a plastic-degrading microorganism screening medium including a medium and an island-like or mesh-like polymer layer formed on the medium is introduced to the present invention, so that it is easy to observe the degradation activity of the polymer layer.

The medium according to an exemplary embodiment may include a solid medium.

The solid medium refers to a medium solidified by adding a component which solidifies a liquid medium without changing the components of the medium. It is preferred that the solidifying agent is a material which is present as a liquid phase in a certain temperature range and becomes a transparent gel within a screening incubation temperature to allow stationary culture of microorganisms.

In addition, the present invention may have a significant effect of solving problems such as precipitation or agglomeration of the plastic powder and complexity of emulsion preparation at once, by providing the plastic-degrading microorganism screening medium.

The island-like polymer layer or the mesh-like polymer layer may be produced by spraying a solution in which plastic is dissolved in a solvent on a commonly used solid medium.

The island-like polymer layer or the mesh-like polymer layer according to an exemplary embodiment of the present invention may be produced by spraying through an atomization device, and the mesh-like polymer layer may be produced by a screen printing method and the like, but is not limited thereto.

By spraying the plastic solution on the solidified solid medium with an atomization device, a plastic-degrading microorganism screening medium in which the plastic solution is formed as the island-like polymer layer or the mesh-like polymer layer on the medium may be produced.

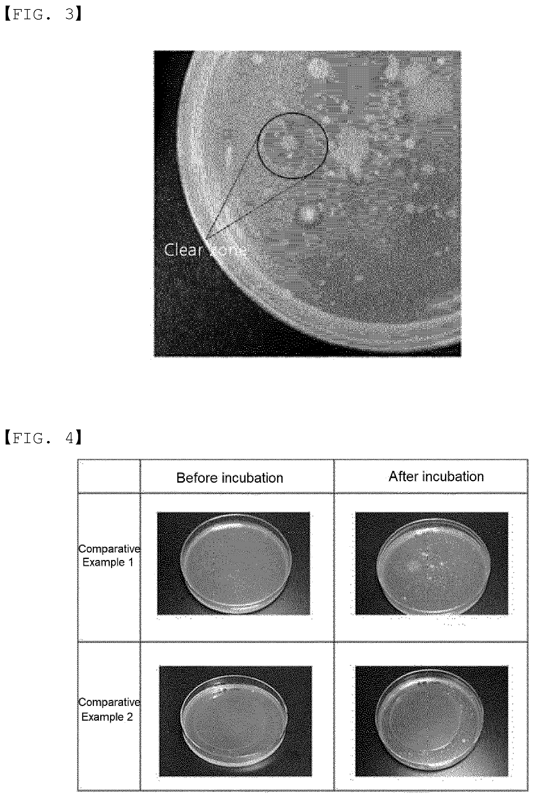

Since the island-like polymer layer or the mesh-like polymer layer is dispersed on the solid medium, a clear zone or a halo zone may be clearly identified by the naked eye when microorganisms are incubated and screened, as shown in the following FIG. 3.

This has a significant effect of screening plastic-degrading microorganisms by the presence of the clear zone or the halo zone, directly by the naked eye, while the production method is simpler than a conventional method of mixing plastic fine powder and a plastic solution emulsion.

The atomization device according to an exemplary embodiment of the present invention may be selected from an atomizer, a sprayer, an air brush, or an electrospinning device, but is not limited thereto as long as the plastic solution may be sprayed.

By spraying the plastic solution on the solid medium with the spraying device, the island-like polymer layer or the mesh-like polymer layer may be simply produced on the solid medium.

The island-like polymer layer according to an exemplary embodiment of the present invention may have a particle diameter of 500 um or less, preferably 0.01 to 500 µm, and more preferably 1 to 500 µm, but is not limited thereto.

By satisfying the particle diameter of the island-like polymer layer, when microorganisms are incubated on the plastic-degrading microorganism screening medium, it may be confirmed that the clear zone or the halo zone is clearly formed.

The mesh-like polymer layer according to an exemplary embodiment of the present invention may have a fiber diameter of 200 um or less, preferably 0.01 to 200 µm, and more preferably 1 to 200 µm, but is not limited thereto.

By satisfying the fiber diameter of the mesh-like polymer layer, when microorganisms are incubated on the plastic-degrading microorganism screening medium, it may be confirmed that the clear zone or the halo zone is clearly formed.

Hereinafter, a method of producing the plastic-degrading microorganism screening medium will be described in detail.

In the present invention, the plastic-degrading microorganism screening medium is produced by a production method including: spraying a plastic solution on a sterilized base solid medium to form an island-like or mesh-like polymer layer on the solid medium.

The solid medium according to an exemplary embodiment may include one or more selected from the group consisting of agar, gelatin, silica gel, serum, and albumin, but is not limited thereto as long as it is a commonly used solid medium.

In addition, the step of producing the medium may include liquefying the medium solidifying agent and sterilizing microorganisms included in a medium composition. In general, liquefaction of the solidifying agent and microbial death may be induced under the conditions of high temperature and pressure.

Specifically, the sterilization at high temperature with pressure may be performed for 10 minutes to 60 minutes under the conditions of a temperature of 100°C to 150°C and a pressure of 10 psi to 50 psi, preferably for 15 minutes to 30 minutes under the conditions of a temperature of 121°C to 135°C and a pressure of 13 psi to 30 psi, but is not limited as long as sterilization is performed.

In addition, inorganic compounds such as metal salts or vitamins and precursors which are further supplemented to the medium may be added to the medium after sterilizing them alone or in combination by an appropriate sterilization method selected from methods such as sterilization at high temperature and high pressure, filter sterilization, and UV sterilization, but are not limited thereto.

The medium which is cooled down after the sterilization is dispensed into a petri dish and solidified under a sterile environment, though not particularly limited thereto, for example, on a sterile experiment bench, and the completed solid medium may be used directly, or stored at 4°C after wrapping the medium in a plastic wrap or an aluminum foil so that the medium is not contaminated and then turning over the medium.

Hereinafter, a step of preparing a plastic solution for atomizing on the solid medium produced above will be described.

Conventionally, a microorganism screening medium was provided by a method of mixing plastic fine powder or a method of mixing with an emulsion prepared with a plastic solution to prepare the medium, but since a clear zone or a halo zone was not clear, it was difficult to screen microorganisms.

In addition, the method of producing a medium by mixing with the emulsion may be changed depending on the conditions of the compounds included in the production of a solid medium.

However, by adopting a method of spraying a solution in which plastic is dissolved on the solid medium, an island-like polymer layer or the mesh-like polymer layer may be provided, regardless of the type of solid medium.

According to an exemplary embodiment of the present invention, the type of plastic in the plastic solution is not limited. As the plastic, polymers ranging from biodegradable polymers such as an aliphatic polyester polymer to commercial polymers such as polyolefin may be used, but are not limited thereto.

Specifically, the plastic may use one or more materials selected from plastics such as polylatic acid, (PLA), polybutylene succinate (PBS), polybutylene succinate adipate (PBSA), polybutylene adipate phthalate (PBAT), polybutylene adipate-co-fumarate (PBAF), poly(caprolactone) (PCL), polyhydroxyalkanoate (PHA), poly-β-hydroxybutyrate (PHB), polystyrene (PS), polyethylene terephthalate (PET), high density polyethylene (HDPE), low density polyethylene (LDPE), polypropylene (PP), polyvinyl chloride (PVC), polyamide, polycarbonate, and polyurethane as a main carbon source, and may include sugars, carbohydrates, or alcohols, such as glucose, sucrose, arabinose, galactose, glycerol, fructose, lactose, maltose, dextrin, glycogen, and mannitol, but is not limited thereto.

As a nitrogen source, an inorganic nitrogen source such as ammonium chloride, ammonium carbonate, ammonium nitrate, ammonium sulfate, and ammonia, or an organic nitrogen source such as amino acid, peptone, tryptone, a yeast extract, a malt extract, a meat extract, a soybean wheat, and a corn immersion liquid may be used separately, or as a mixture. Potassium dihydrogen phosphate, dipotassium hydrogen phosphate, or a sodium-containing salt corresponding thereto may be used as a phosphorus source. In addition, as the inorganic compound, sodium chloride, potassium chloride, iron chloride, magnesium sulfate, iron sulfate, manganese sulfate, calcium carbonate, and the like may be used, and vitamins, appropriate precursor, and the like may be included in addition to the above materials.

The solvent of the plastic solution is not limited as long as it is a solvent dissolving the plastic.

Specifically, the solvent may be any one or a mixture of two or more selected from chloroform, trifluoroacetic acid, methyl chloride, carbon tetrachloride, formic acid, 1,1,1,3,3,3-hexafluoro-2-propanol (HFIP), benzene, carbon disulfide, cyclohexanone, dioxane, ethyl acetate, ethylbenzene, methyl ethyl ketone (MEK) N-methyl-2-pyrrolidone (NMP), tetrahydrofuran (THF), acetonitrile, 1,1,2-trichloroethane, dichloroacetic acid, ethanol, methanol, isopropyl alcohol (IPA), acetone, 1-propanol, distilled water, and the like, but is not limited thereto.

The plastic solution according to an exemplary embodiment of the present invention may have a solid content of 0.01 to 20 wt%, but is not limited thereto as long as the island-like polymer layer or the mesh-like polymer layer is formed well on the solid medium.

Hereinafter, a step of spraying the plastic solution prepared on the medium produced will be described.

The spraying method may be performed by spraying with compressed air, electrospraying, or combined spraying of the two methods, but is not limited thereto.

The method used in the spraying method may be spraying using coaxial double or multiple nozzles, an atomizer, a sprayer, an air brush, or an electrospinning method.

The solid content of the plastic solution is not limited as long as the island-like polymer layer or the mesh-like polymer is provided.

The pneumatic spraying method according to an exemplary embodiment of the present invention is not limited, but specifically, may be spraying equipment including a nozzle part, a quantitative discharge device, and an air compressor, and
the island-like polymer layer or the mesh-like polymer layer may be applied on the surface of the solid medium by a method of adding air pressure to the plastic solution which is quantitatively discharged from the spraying equipment.

In the present invention, the pressure of the added air may be 5 to 100 psi, specifically 5 to 30 psi, but is not limited thereto.

As the air pressure in the above range is added to the discharged plastic solution and the solution is sprayed, an effect of evenly applying on the solid medium may be exhibited, and particles may be broken into small pieces to impart a large surface area.

In addition, according to another exemplary embodiment of the present invention, the plastic-degrading microorganism screening medium may be produced by an electrospraying method.

Specifically, high voltage is applied to a quantitatively discharging plastic solution, using spraying equipment including a nozzle part which discharges the plastic solution, a quantitative discharge device, a high-voltage generation device, and a current collector, thereby applying an island-like polymer layer or a mesh-like polymer layer on the solid medium.

In the present invention, the high voltage applied to the nozzle in the electrospraying method may be 1 to 60 kV, specifically 2 to 10 kV, but is not limited thereto.

When the voltage range is satisfied, a dispersion degree of the particles of the polymer layer may be better by a repulsive force due to an electric charge accumulated in the plastic solution, during the production of the island-like polymer layer.

In the present invention, in the methods of pneumatic spraying, electrospraying, or combined spraying, a discharge speed of the plastic solution is not particularly limited, and may be 0.5 to 100 mL/h, specifically 1 to 20 mL/h, but is not limited thereto.

The diameter of the plastic discharge nozzle of the present invention may be 0.1 to10 mm, specifically 0.2 to 0.5 mm, and may have a cylindrical structure, but is not limited thereto.

Spraying of the plastic solution through the spraying method in the above range may not interfere with the direct supply of nutrients to the growth of microorganisms.

In addition, it has an effect of more uniformly forming polymer particles or a polymer fiber layer (mesh layer) on a medium surface, and has an excellent effect of solvent volatilization in a solution.

Next, a base medium on which the plastic solution is sprayed in the present invention may be a medium smeared with a microorganism culture liquid or a liquid sample, and when the base medium is a medium in a sterile state, a method of inoculation on a plastic-degrading microorganism screening medium produced by spraying a plastic solution by a method of atomizing a liquid sample or a microorganism culture liquid may be used, but the present invention is not limited thereto.

It is preferred that the incubation temperature is set to a temperature at which the medium maintains a solidified state so that it does not drip, and may be specifically 20°C to 50°C, but is not limited thereto. The incubation period of the screening medium may continue until a desired aspect appears.

Hereinafter, the present invention will be described in more detail with reference to the examples and the comparative examples. However, the following examples and comparative examples are only one example for describing the present invention in more detail, and do not limit the present invention in any way.

### [Production Example 1]

A plastic solution was applied on an LB agar medium (Luria-Bertani broth) by a pneumatic spraying method to produce a plastic-degrading microorganism screening medium. The medium was produced by the following method. 10 g/L of tryptone, 10 g/L of sodium chloride (NaCl), 5 g/L of a yeast extract, and 15 g/L of agar were mixed. The mixture was sterilized at high temperature with pressure, and about 10 mL to 20 mL of the mixture was poured into a petri dish of 90 mm×15 mm (diameter×height) on a sterile work bench, and was solidified. The plastic solution was prepared by dissolving 1 g of polylactic acid (PLA, Natureworks LLC, Minnetonka, MN, USA) in 99 g of chloroform to produce a 1 wt% plastic solution.

The agar medium produced above was placed on a ground in a vertical direction to the nozzle of spraying equipment, and the plastic solution was sprayed thereon using a coaxial double nozzle. At this time, the coaxial double nozzle was a nozzle including an internal nozzle having an inner diameter of 0.26 mm and an outer diameter of 0.50 mm and an external nozzle having an inner diameter of 0.86 mm and an outer diameter of 1.26 mm. The solution was sprayed at a discharge speed of the plastic solution in the internal nozzle of 12 mL/h and an air pressure applied to the external nozzle of 20 psi, thereby producing a plastic-degrading microorganism screening medium. A spacing between the lower portion of the internal nozzle and the agar medium was 25 cm and the amount of sprayed PLA was 15 mg.

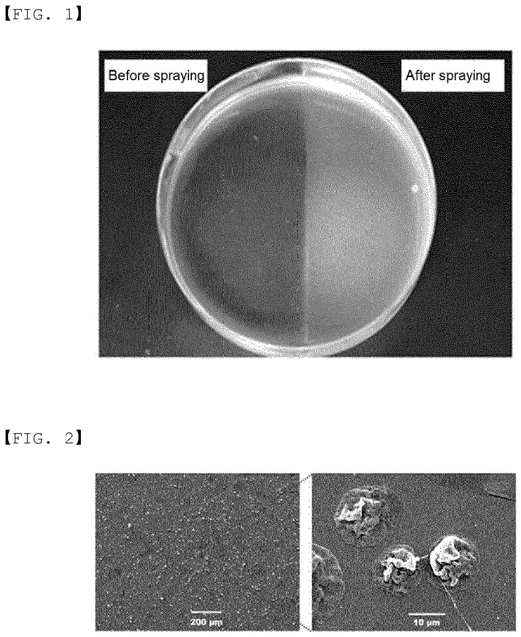

As seen in FIG. 1, it was confirmed that a translucent aspect was shown due to the formation of a plastic layer, as compared with the agar medium on which the plastic solution was not sprayed. As a result of observation with an electron microscope, as seen in FIG. 2, it was confirmed that PLA fine particles of 500 um or less were uniformly distributed in the medium.

### [Production Example 2]

The solid medium was produced in the same manner as in Production Example 1, except that a compression atomizer was used instead of a pneumatic spraying method. PLA fine particles of 500 um or less were uniformly distributed in the medium, as in Production Example 1, thereby producing a translucent solid medium.

### [Production Example 3]

An LB solid medium was produced in the same manner as in Production Example 1, and plastic fine particles were applied on the LB solid medium by an electrospinning method, not the pneumatic spraying method.

The plastic solution used in the electrospinning method was prepared by dissolving 1 g of polylactic acid (PLA, Natureworks LLC, Minnetonka, MN, USA) in 99 g of chloroform to produce a 1 wt% plastic solution.

The LB solid medium produced above was placed on the ground in the vertical direction to the nozzle of the spraying equipment.

The discharge speed of the solution from a single nozzle was 2 mL/h, and a voltage of 6 kV was applied in a high-voltage generation device connected to the nozzle to spray the solution on the LB solid medium, thereby producing a plastic-degrading microorganism screening medium.

At this time, the single nozzle was a nozzle having an inner diameter of 0.26 mm and an outer diameter of 0.50 mm. A spacing between the lower portion of the nozzle and the agar medium was 12 cm and the amount of sprayed PLA was 10 mg.

PLA fine particles of 500 um or less were uniformly distributed in the medium, as in Example 1, thereby producing a translucent solid medium.

### [Production Example 4]

Spraying was performed on the LB solid medium at the concentration of the electrospinning solution of 4 wt%, not 1 wt%, in the same manner as in Example 3, thereby applying plastic fine fiber. A PLA fine fiber phase having a fiber bundle diameter of 200 um or less was uniformly distributed in the medium, thereby producing a translucent solid medium.

### [Production Example 5]

200 mg of crushed polylactic acid (PLA, Hebei Runk Tech Co., Ltd, Hebei, China) of 500 um to 1000 um was mixed with 100 mL of a liquefied medium, and the mixture was poured into a petri dish at 15 ml each and was solidified. It was confirmed that the crushed PLA particles settled below the solid medium, due to the visually identifiable size.

### [Production Example 6]

Before the medium was poured into the petri dish and solidified, a PLA (NatureWorks LCC) film having a thickness of 180 um was placed thereon and was solidified.

### [Example 1]

In order to screen microorganisms which may degrade plastic from the solid medium produced in Production Example 1, a sample was collected from a sewage treatment plant sludge and inoculated on a screening medium, which was incubated at 30±2°C in a laboratory.

In the screening of microorganisms which may degrade plastics in the present invention, when a clear zone or a halo zone was shown around a microorganism colony, it was determined that the microorganisms had plastic degradation activity.

### [Example 2]

The process was performed in the same manner as in Example 1, except that the solid medium of Production Example 2 was used instead of the solid medium of Production Example 1.

In the screening of microorganisms which may degrade plastics in the present invention, when a clear zone or a halo zone was shown around a microorganism colony, it was determined that the microorganisms had plastic degradation activity. The results are shown in Table 1.

### [Example 3]

The process was performed in the same manner as in Example 1, except that the solid medium of Production Example 3 was used instead of the solid medium of Production Example 1.

In the screening of microorganisms which may degrade plastics in the present invention, when a clear zone or a halo zone was shown around a microorganism colony, it was determined that the microorganisms had plastic degradation activity. The results are shown in Table 1.

### [Example 4]

The process was performed in the same manner as in Example 1, except that the solid medium of Production Example 4 was used instead of the solid medium of Production Example 1.

In the screening of microorganisms which may degrade plastics in the present invention, when a clear zone or a halo zone was shown around a microorganism colony, it was determined that the microorganisms had plastic degradation activity. The results are shown in Table 1.

### [Comparative Example 1]

The process was performed in the same manner as in Example 1, except that the solid medium of Production Example 5 was used instead of the solid medium of Production Example 1.

In the screening of microorganisms which may degrade plastics in the present invention, when a clear zone or a halo zone was shown around a microorganism colony, it was determined that the microorganisms had plastic degradation activity. The results are shown in Table 1.

### [Comparative Example 2]

The process was performed in the same manner as in Example 1, except that the solid medium of Production Example 6 was used instead of the solid medium of Production Example 1. The results are shown in Table 1.

In the screening of microorganisms which may degrade plastics in the present invention, when a clear zone or a halo zone was shown around a microorganism colony, it was determined that the microorganisms had plastic degradation activity.

**[Table 1]**

| | Clear zone or halo zone |
|---|---|
| Example 1 | Identifiable |
| Example 2 | Identifiable |
| Example 3 | Identifiable |
| Example 4 | Identifiable |
| Comparative Example 1 | Difficult to identify |
| Comparative Example 2 | Impossible to identify |

As shown in FIG. 3, it was confirmed in Example 1 that there were microorganisms forming a clear zone around a colony due to the activity degrading plastic particles on the surface, among the microorganisms incubated on the screening medium.

In addition, as shown in FIG. 4, in Comparative Example 1, it was difficult to visually identify a clear zone, and in Comparative Example 2, a clear zone did not occur on the film, so that it was impossible to identify the microorganisms degrading plastics themselves.

In the above examples, since a visual change by plastic degradation was clearly shown, it was easy to perform screening of microorganisms and separation of a single colony. However, in the comparative examples, the visual change was unclear and the screening degree of microorganisms was low.

In summary, the present inventors were able to produce a screening plate for screening plastic-degrading microorganisms, effectively distinguish microorganisms having plastic degradation activity with the naked eye through microorganism incubation, and perform pure separation of a single colony. This means that the microorganisms having plastic degradation activity may be screened using the plate.

As the industrial applicability of the technology, for example, a solid medium kit in which plastics such as PLA are distributed in a fine phase may be manufactured and sold, and the kit may be used as a basic material for screening, studying, and commercializing plastic-degrading microorganisms present in the nature.

Hereinabove, although the present invention has been described by specific matters, limited exemplary embodiments, and drawings, they have been provided only for assisting the entire understanding of the present invention, and the present invention is not limited to the exemplary embodiments, and various modifications and changes may be made by those skilled in the art to which the present invention pertains from the description.

Therefore, the spirit of the present invention should not be limited to the above-described exemplary embodiments, and the following claims as well as all modified equally or equivalently to the claims are intended to fall within the scope and spirit of the invention.

## Claims

1. A plastic-degrading microorganism screening medium comprising:
a medium and
an island-like or mesh-like polymer layer formed on the medium.

2. The plastic-degrading microorganism screening medium of claim 1, wherein the plastic-degrading microorganism screening medium allows observation of degradation activity of the polymer layer, by applying microorganisms degrading a polymer on the polymer layer.

3. The plastic-degrading microorganism screening medium of claim 2, wherein the island-like polymer layer or the mesh-like polymer layer is produced by spraying through an atomization device.

4. The plastic-degrading microorganism screening medium of claim 3, wherein the atomization device is selected from an atomizer, a sprayer, an air brush, or an electrospinning device.

5. The plastic-degrading microorganism screening medium of claim 2, wherein the island-like polymer layer has a particle diameter of 500 um or less, and the mesh-like polymer layer has a fiber diameter of 200 um or less.

6. A method of producing a plastic-degrading microorganism screening medium, the method comprising: spraying a plastic solution on a sterilized base solid medium to form an island-like or mesh-like polymer layer on the solid medium.

7. The method of producing a plastic-degrading microorganism screening medium of claim 6, wherein the solid medium includes one or more selected from the group consisting of agar, gelatin, silica gel, serum, and albumin.

8. The method of producing a plastic-degrading microorganism screening medium of claim 6, wherein the plastic solution has a solid content of 0.01 to 20 wt%.

9. The method of producing a plastic-degrading microorganism screening medium of claim 6, wherein the spraying is performed using an electrospinning device or spraying equipment including a coaxial double or multiple nozzle part which discharges the plastic solution and an air compression part.

10. The method of producing a plastic-degrading microorganism screening medium of claim 9, wherein a voltage of the electrospinning device is 1 to 60 kV, and a discharge speed of the plastic solution is 0.5 to 100 ml/h.
